# EUROPEAN PATENT APPLICATION

(11) **EP 2 719 276 A1**
(43) Date of publication of application: **16.04.2014**
(21) Application number: 12796796.6
(22) Date of filing: 08.06.2012
(51) Int. Cl.: A01H 1/08, A01G 7/06

(54) **METHOD FOR PRODUCING PARA RUBBER TREE POLYPLOID**

(30) Priority: 10.06.2011 JP 2011130381; 10.06.2011 JP 2011130382
(71) Applicant: Bridgestone Corporation, Tokyo 104-8340 (JP); The Agency For The Assessment And Application Of Technology, Jakarta 10340 (ID)
(72) Inventor: WATANABE Norie, Kodaira-shi Tokyo 187-8531 (JP)
(74) Representative: Waldren, Robin Michael
(86) International application number: PCT/JP2012/064780
(87) International publication number: WO 2012/169613

(57) **Abstract**

An object of the present invention is to provide a method for efficiently producing polyploids in para rubber trees that are extremely useful for breeding new varieties. The method of the present invention is characterized by carrying out colchicine treatment on para rubber tree (Hevea brasiliensis) seeds after rooting. In the present invention, the seeds are preferably seeds that have rooted but have not yet germinated. In addition, colchicine treatment is preferably carried out by allowing the seeds to contact a colchicine solution having a colchicine concentration of 400 ppm to 800 ppm.

## Description

### TECHNICAL FIELD

The present invention relates to a method for efficiently producing polyploids of para rubber trees.

### BACKGROUND ART

Natural rubber is an elastic polymer this is widely used in large quantities in various applications as the primary raw material of rubber products. Natural rubber is produced by collecting latex, which is secreted by latex-producing plants such as rubber trees, and subjecting the latex to desired processing. Consequently, rubber trees, and particularly para rubber trees (Hevea brasiliensis), are planted commercially mainly in tropical countries such as Thailand, Malaysia and Indonesia.

Accompanying progress made in the field of genetic engineering in recent years, it has become possible to modify the traits of naturally-occurring plants by inserting preferable foreign genes. In the field of natural rubber production as well, research is being conducted on methods for producing plants having desirable traits, such as plants able to produce latex of higher quality or plants able to produce larger amounts of latex, by genetically modifying latex-producing plants. Although an example of a particularly preferable method consists of molecular breeding by genetic recombination, it is necessary to go through processes consisting of redifferentiation and gene insertion in order to obtain genetically recombinant plants.

Examples of methods mainly used to insert genes into plants include a method consisting of inserting a gene by infecting plant cells with Agrobacterium species, a type of plant pathogen (Agrobacterium method), and a method consisting of shooting gene-laden gold particles into plant cells with a particle gun (particle gun method) (see, for example, Patent Document 1).

According to the Agrobacterium method, there have been several successful examples of producing transformants of the para rubber tree. For example, a method has been disclosed by which transformed para rubber trees are produced by inserting a desired gene into a callus derived from the anther of para rubber tree using an Agrobacterium bacterial vector and regenerating a plant from this plant tissue (see, for example, Patent Document 2). In addition, the obtaining of a transformant from a callus derived from the integument of para rubber tree using the Agrobacterium method has also been reported (see, for example, Non-Patent Document 1).

However, latex-producing plants such as the para rubber tree have problems such as greater difficulty in inserting genes and extremely poor gene insertion efficiency in comparison with other types of plants. In the aforementioned examples as well, although studies have been conducted on increasing gene insertion efficiency by adjusting the bacterial concentration of the Agrobacterium used when infecting the callus, an adequate level of gene insertion efficiency has yet to be achieved.

In addition, there are also cases in which traits can be altered by increasing the number of chromosomes of an individual. Normally, somatic cells are diploids having two sets of chromosomes required for survival, while germ cells are haploids having one set of chromosomes. In general, individuals having three or more sets of chromosomes (namely, triploid or greater individuals) are referred to as polyploids. In plants, polyploids tend to have larger fruit and other tissue than diploids and also tend to not form seeds.

In this manner, breeding of polyploids has been conventionally carried out in the field of plant selective breeding in order to obtain new varieties of plants having new traits without introducing a foreign gene. For example, polyploids in which the number of chromosomes has doubled can be produced by immersing seeds or other plant tissue in a colchicine solution. Since colchicine inhibits spindle formation during cell division, cell division is inhibited in plants treated with colchicine. On the other hand, cells in which the number of chromosomes has been doubled are formed since chromosome disjunction is inhibited.

### Prior Art Documents

### Patent Documents

Patent Document 1: Japanese Unexamined Patent Application, First Publication No. 2005-130815
Patent Document 2: Japanese Patent Publication No. 3289021 Non-Patent Documents
Non-Patent Document 1: Blanc, et al., Plant Cell Report, 2006, Vol. 24, pp. 724-733

### DISCLOSURE OF THE INVENTION

### Problems to be Solved by the Invention

Although breeding of polyploids is important in terms of producing useful new varieties of plants having more desirable traits, the conditions of colchicine treatment vary considerably according to the type of plant. Consequently, it is necessary to determine preferable treatment conditions through repeated trial and error for each type of plant, and a method for producing effective polyploids in para rubber trees has yet to be established.

With the foregoing in view, the present invention relates to a method for efficiently producing polyploids in para rubber trees that are extremely useful for breeding new varieties. Means for Solving the Problems

As a result of conducting extensive studies to solve the aforementioned problems, the inventors of the present invention found that, by carrying out colchicine treatment on para rubber tree seeds after they have rooted, polyploids can be obtained at a higher probability than in the past, thereby leading to completion of the present invention.

Namely, the present invention is composed in the manner indicated below.
(1) A method for producing polyploids of para rubber tree, comprising: a colchicine treatment step in which colchicine treatment is carried out on seeds of para rubber tree (Hevea brasiliensis) that have rooted.
(2) The method for producing polyploids of para rubber tree described in (1) above, comprising: a rooting step wherein para rubber tree seeds are allowed to root by impregnating with water at 15°C to 25°C prior to the colchicine treatment step.
(3) The method for producing polyploids of para rubber tree described in (1) above, wherein the seeds subjected to the colchicine treatment step are seeds that have rooted but have not yet germinated.
(4) The method for producing polyploids of para rubber tree described in (1) above, wherein the colchicine treatment step is a step in which the seeds are contacted with a colchicine solution having a colchicine concentration of 400 ppm to 800 ppm.
(5) The method for producing polyploids of para rubber tree described in (4) above, wherein the colchicine treatment step is a step in which the seeds are contacted with the colchicine solution for 40 hours to 90 hours.
(6) The method for producing polyploids of para rubber tree described in (5) above, wherein the colchicine treatment step is a step in which the seeds are contacted with a colchicine solution having a colchicine concentration of 600 ppm to 800 ppm for 60 hours to 80 hours.
(7) The method for producing polyploids of para rubber tree described in any of (1) to (6) above, wherein the seeds are allowed to germinate and grow following the colchicine treatment step.

### Effects of the Invention

Polyploids can be produced extremely efficiently by using the method for producing polyploids of para rubber tree of the present invention. In particular, the present invention allows mature polyploids to be easily obtained by allowing seeds treated with colchicine to be grown in the same manner as diploid seeds.

### BRIEF DESCRIPTION OF THE DRAWINGS

FIG. 1 is a drawing showing the results of analyzing diploids by flow cytometry in Example 1.
FIG. 2 is a drawing showing the results of analyzing tetraploids by flow cytometry in Example 1.
FIG. 3 is a drawing showing the results of analyzing chimera by flow cytometry in Example 1.

### EMBODIMENTS FOR CARRYING OUT THE INVENTION

Colchicine treatment is typically carried out by treating not only seeds, but also tissue such as shoots or leaves, as well as calluses obtained by dedifferentiation treatment. For example, in the case of carrying out colchicine treatment on tissue such as a plant shoot, polyploidal plants can be regenerated by isolating polyploidal cells from the tissue and allowing to root and the like. In addition, in the case of treating calluses with colchicine, polyploidal plants can be similarly regenerated from the calluses by normal methods. However, the regeneration of plants from a callus or tissue from a portion of a plant involves a complex process, and is also expensive since it requires suitable plant hormones and the like. In contrast, in the case of treating seeds with colchicine, polyploidal plants can be easily obtained by cultivating the seeds in the same manner as untreated seeds.

However, hardly any polyploids are obtained simply by immersing para rubber tree seeds in a colchicine solution. The inventors of the present invention believed that, since the seeds of para rubber trees are covered by a hard husk, the colchicine is unable to adequately penetrate inside the seeds, thereby preventing colchicine treatment from being effectively carried out on para rubber tree seeds. Therefore, when a portion of the seed husk was cut open, although the colchicine was able to adequately penetrate into the seeds, the seeds did not germinate and polyploids were unable to be obtained.

The method for producing polyploids of para rubber tree of the present invention (to also be referred to as the "polyploid production method") is characterized by carrying out colchicine treatment on seeds after rooting. As a result of carrying out colchicine treatment on seeds after rooting, it was found that colchicine treatment can be effectively carried out on the seeds without impairing seed germination or growth, and that polyploidal plants can be easily obtained by cultivating the treated seeds in the same manner as untreated seeds. Although the reason for being able to efficiently obtain polyploids, in which the number of chromosomes is double that of seeds prior to treatment, by treating seeds with colchicine after rooting is unclear, it is presumed that the colchicine is able to efficiently penetrate into the seeds through those portions of the seeds where roots have protruded from the hard husk.

There are no particular limitations on the seeds subjected to colchicine treatment in the polyploid production method of the present invention provided they have rooted, or in other words, can be visually confirmed to have roots on the outside thereof. In the present invention, seeds at an early stage after rooting such as seeds prior to germination are more preferable. By carrying out colchicine treatment on seeds in an early stage of growth, plants can be obtained in which nearly all cells that compose the plant are polyploidal.

There are no particular limitations on the method used to allow the seeds to root, and any method may be used that is normally used when allowing seeds to root. For example, seeds can be allowed to root by allowing the seeds to stand undisturbed for several days to two weeks on a support such as a piece of gauze or tissue adequately soaked with water at 20°C to 30°C. The seeds may also be wrapped in a support soaked with water. In the present invention, para rubber tree seeds are allowed to root by impregnating with water at 15°C to 25°C. Although there are no particular limitations on the duration for which the seeds are impregnated with water provided it is an adequate duration for allowing the seeds to root, the seeds are preferably subjected to the subsequent colchicine treatment after they have rooted but prior to germination.

There are no particular limitations on the seeds subjected to colchicine treatment in the polyploid production method of the present invention provided they are para rubber tree seeds, and may be diploid para rubber tree seeds present in nature, variants in which a portion of a gene has been deleted by gene recombination technology and the like, or genetic recombinants incorporating a foreign gene and the like. In addition, the seeds may be a polyploid such as a triploid or tetraploid. For example, a para rubber tree can be obtained in which the number of chromosomes has increased six-fold by carrying out the polyploid production method of the present invention on triploid para rubber tree seeds.

Colchicine treatment is carried out by contacting seeds with a colchicine solution after they have rooted. There are no particular limitations on the method used to contact the seeds with the colchicine solution, and the seeds may be immersed in the colchicine solution, the seeds may be placed undisturbed on a support such as a piece of gauze soaked with the colchicine solution, or the seeds may be wrapped in that support. Furthermore, in the case of using a support soaked with a colchicine solution, the root portions of the seeds are preferably contacted with the colchicine solution.

There are no particular limitations on the temperature during colchicine treatment provided the temperature does not impair subsequent seed growth. In addition, colchicine treatment may be carried out in a temperature-controlled environment or in an environment such as a greenhouse that is not provided with any particular temperature control. In the present invention, colchicine treatment can be carried out at, for example, a temperature of 0°C to 35°C and preferably 20°C to 30°C. Colchicine treatment may also be preferably carried out at room temperature for the sake of convenience.

The concentration of colchicine in the colchicine solution can be suitably determined in consideration of such factors as the method and temperature used to carry out colchicine treatment, treatment time and the like. In the case the colchicine concentration is excessively low, colchicine is unable to adequately penetrate into the seeds and chromosomes are unable to double. On the other hand, in the case the colchicine concentration is excessively high, there is the risk of occurrence of malformation. In the present invention, the colchicine concentration in the colchicine solution that contacts the seeds is preferably 400 ppm to 800 ppm and more preferably 600 ppm to 800 ppm.

The amount of time the seeds are allowed to contact the colchicine solution (treatment time) can be suitably determined in consideration of such factors as the method and temperature used to carry out colchicine treatment, the concentration of colchicine in the colchicine solution and the like. In the case the treatment time is excessively short, colchicine is unable to adequately penetrate into the seeds and chromosomes are unable to double. On the other hand, in the case the treatment time is excessively long, there is the risk of occurrence of malformation. In the present invention, the amount of time the seeds are allowed to contact the colchicine solution at room temperature is preferably 40 hours to 90 hours and more preferably 60 hours to 80 hours.

In the polyploid production method of the present invention, colchicine treatment is preferably carried out by immersing rooted seeds in a colchicine solution having a concentration of 400 ppm to 800 ppm, preferably 600 ppm to 800 ppm, and more preferably 600 ppm, for 40 hours to 90 hours, preferably 60 hours to 80 hours, and more preferably 72 hours, in a temperature-controlled environment at 20°C to 30°C or a non-controlled environment.

Following colchicine treatment, the seeds are washed with distilled water and the like to remove residual colchicine followed by cultivating in accordance with ordinary methods in the same manner as untreated seeds. For example, after allowing the seeds to germinate by placing undisturbed for several days on a substrate such as a piece of gauze or tissue adequately soaked with water at 20°C to 30°C, polyploids are grown by transplanting the seeds into cultivation medium followed by cultivating while providing adequate water.

For example, tetraploid para rubber trees can be produced by carrying out the polyploid production method of the present invention on diploid para rubber tree seeds. Triploid para rubber trees can be produced by breeding these tetraploids and crossing them with diploids.

### Examples

Although the following provides a more detailed explanation of the present invention by indicating examples thereof, the present invention is not limited to the following examples.

### [Example 1]

Naturally-occurring diploid para rubber tree seeds were placed on a piece of gauze soaked with water for several days to one week at room temperature (about 20°C to 30°) and allowed to root. Those seeds for which rooting was confirmed visually but which had not yet germinated were immersed in a colchicine solution having a colchicine concentration of 200 ppm to 800 ppm for 2 days to 5 days. Subsequently, the seeds were transplanting into a planter after washing with distilled water.

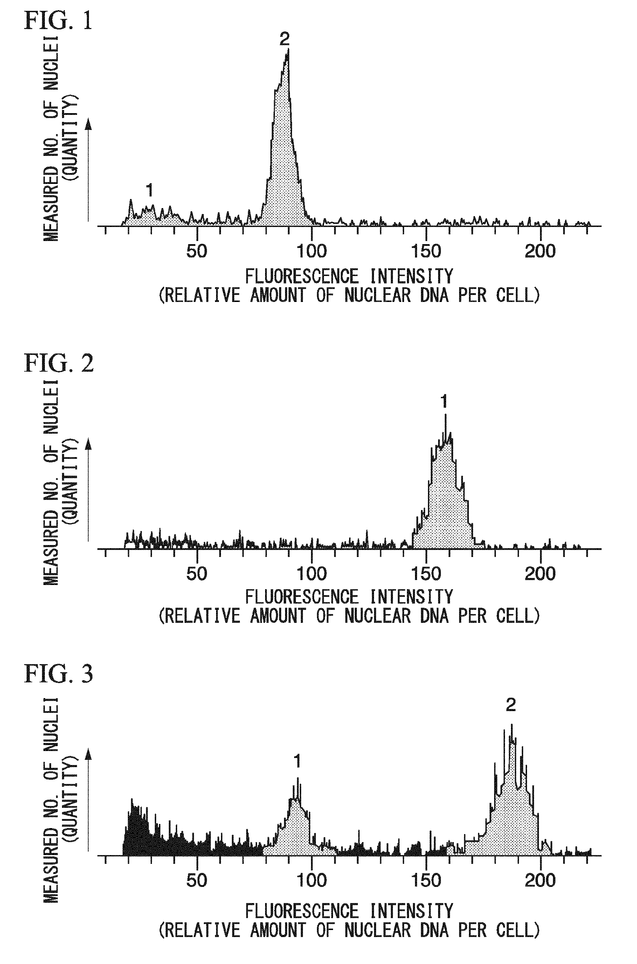

Three leaves were collected from the grown plants, and the number of chromosomes (ploidy) was confirmed by flow cytometry, and the plants were confirmed to be diploids, tetraploids or chimera containing a mixture of diploid cells and tetraploid cells. The results are shown in FIGS. 1 to 3 and Table 1.

**[Table 1]**

| Colchicine concentration | Treatment time | No. of polyploids |
|---|---|---|
| ppm | hr | % |
| 400 | 72 | 2 |
| 600 | 48 | 7 |
| | 72 | 15 |
| 800 | 48 | 6 |
| | 72 | 10 |

As shown in Table 1, polyploids in which the original number of chromosomes had doubled were able to be efficiently produced by immersing rooted seeds in a colchicine solution having a colchicine concentration of 400 ppm to 800 ppm, preferably 600 ppm to 800 ppm, and more preferably 600 ppm for 40 hours to 90 hours, preferably 60 hours to 80 hours, and more preferably 72 hours.

### INDUSTRIAL APPLICABILITY

The method for producing polyploids of para rubber tree of the present invention is useful in the field of selective breeding of para rubber trees and the field of natural rubber production since it allows para rubber tree polyploids to be efficiently produced.

## Claims

1. A method for producing polyploids of para rubber tree, comprising:
a colchicine treatment step in which colchicine treatment is carried out on seeds of para rubber tree (Hevea brasiliensis) that have rooted.

2. The method for producing polyploids of para rubber tree according to claim 1, further comprising:
a rooting step wherein para rubber tree seeds are allowed to root by impregnating with water at 15°C to 25°C prior to the colchicine treatment step.

3. The method for producing polyploids of para rubber tree according to claim 1, wherein the seeds subjected to the colchicine treatment step are seeds that have rooted but have not yet germinated.

4. The method for producing polyploids of para rubber tree according to claim 1, wherein the colchicine treatment step is a step in which the seeds are contacted with a colchicine solution having a colchicine concentration of 400 ppm to 800 ppm.

5. The method for producing polyploids of para rubber tree according to claim 4, wherein the colchicine treatment step is a step in which the seeds are contacted with the colchicine solution for 40 hours to 90 hours.

6. The method for producing polyploids of para rubber tree according to claim 5, wherein the colchicine treatment step is a step in which the seeds are contacted with a colchicine solution having a colchicine concentration of 600 ppm to 800 ppm for 60 hours to 80 hours.

7. The method for producing polyploids of para rubber tree according to claim 1, wherein the seeds are allowed to germinate and grow following the colchicine treatment step.
